Europäisches Patentamt

European Patent Office

Office européen des brevets

(19)

(11) Publication number : **0 650 730 A1**

# EUROPEAN PATENT APPLICATION

(12)

(21) Application number : 94307118.3

(22) Date of filing : 29.09.94

(51) Int. Cl.$^6$ : **A61K 31/71,** A61K 9/08,
A61K 47/00, A61K 47/10,
A61K 47/16

(30) Priority : 30.09.93 US 129524

(43) Date of publication of application :
03.05.95 Bulletin 95/18

(84) Designated Contracting States :
AT BE CH DE DK ES FR GB GR IE IT LI LU NL
PT SE

(71) Applicant : **AMERICAN HOME PRODUCTS
CORPORATION
Five Giralda Farms
Madison, New Jersey 07940-0874 (US)**

(72) Inventor : **Waranis, Robert Paul
9753 Route 9,
Chazy
Clinton County, New York (US)**
Inventor : **Leonard, Thomas Waymond
34 Trafalgar Drive,
Plattsburgh
Clinton County, New York (US)**

(74) Representative : **Wileman, David Francis Dr. et
al
c/o Wyeth Laboratories
Huntercombe Lane South
Taplow Maidenhead Berkshire SL6 OPH (GB)**

(54) Rapamycin formulations for oral administration.

(57)   The present invention comprises novel oral rapamycin formulations which have, per 100 ml of formulation, from about 0.05 to about 10.0 grams of rapamycin, from about 0.1 to about 25 ml of N,N-dimethylacetamide, from about 0.1 to about 10 ml of surfactant, and from about 65 to about 99.8 ml of PEG 400.

EP 0 650 730 A1

This invention relates to formulations containing rapamycin, or pharmaceutically acceptable salts of rapamycin, which are useful in oral administrations for inducing immunosuppression and for treating transplantation rejection, host vs. graft disease, autoimmune diseases, diseases of inflammation, solid tumors, fungal infections, adult T-cell leukemia/lymphomas and hyperproliferative vascular disorders.

Rapamycin is a macrolide antibiotic produced by *Streptomyces hygroscopicus* which was discovered first for its properties as an antifungal agent. It adversely affects the growth of fungi such as *Candida albicans* and *Microsporum gypseum*. Rapamycin, its preparation and its antibiotic activity were described in U.S. Patent No. 3,929,992, issued December 30, 1975 to Surendra Sehgal et al. In 1977 Martel, R. R. et al. reported on immunosuppressive properties of rapamycin against experimental allergic encephalitis and adjuvant arthritis in the Canadian Journal of Physiological Pharmacology, 55, 48-51 (1977). In 1989, Calne, R. Y. et al. in Lancet, 1989, no. 2, p. 227 and Morris, R. E. and Meiser, B. M. in Medicinal Science Research, 1989, No. 17, P. 609-10, separately reported on the effectiveness of rapamycin in inhibiting rejection *in vivo* in allograft transplantation. Numerous articles have followed describing the immunosuppressive and rejection inhibiting properties of rapamycin, and clinical investigation has begun for the use of rapamycin in inhibiting rejection in transplantation in man.

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

The immunosuppressive effects of rapamycin have been disclosed in FASEB 3, 3411 (1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); R. Y. Calne et al., Lancet 1183 (1978); and U.S. Patent 5,100,899].

Rapamycin has also been shown to be useful in preventing or treating systemic lupus erythematosus [U.S. Patent 5,078,999], pulmonary inflammation [U.S. Patent 5,080,899], insulin dependent diabetes mellitus [Fifth Int. Conf. Inflamm. Res. Assoc. 121 (Abstract), (1990)], and smooth muscle cell proliferation and intimal thickening following vascular injury [Morris, R. J. Heart Lung Transplant 11 (pt. 2): 197 (1992)].

Mono- and diacylated derivatives of rapamycin (esterified at the 28 and 43 positions) have been shown to be useful as antifungal agents (U.S. Patent 4,316,885) and used to make water soluble prodrugs of rapamycin (U.S. Patent 4,650,803). Recently, the numbering convention for rapamycin has been changed; therefore according to Chemical Abstracts nomenclature, the esters described above would be at the 31- and 42-positions. U.S. Patent 5,118,678 discloses carbamates of rapamycin that are useful as immunosuppressive, anti-inflammatory, antifungal, and antitumor agents. U. S. Patent 5,100,883 discloses fluorinated esters of rapamycin. U. S. Patent 5,118,677 discloses amide esters of rapamycin. U. S. Patent 5,130,307 discloses aminoesters of rapamycin. U. S. Patent 5,117,203 discloses sulfonates and sulfamates of rapamycin. U. S. Patent 5,194,447 discloses sulfonylcarbamates of rapamycin.

U.S. Patent No. 5,100,899 (Calne) discloses methods of inhibiting transplant rejection in mammals using rapamycin and derivatives and prodrugs thereof. Other chemotherapeutic agents listed for use with rapamycin are azathioprine, corticosteroids, cyclosporen (and cyclosporin A), and FK-506, or any combination thereof.

The primary immunosuppressive agent presently used for inhibiting rejection in the allograft transplantation of organs in man is cyclosporine (Sandimmune®). Cyclosporine is a cyclic polypeptide consisting of 11 amino acids. The intravenous injectable formulation of Sandimmune® (IV) is a sterile ampul containing, per ml, 50 mg of cyclosporine, 650 mg of Cremophor® EL and alcohol Ph Helv. (32.9% by volume) (under nitrogen). For administration this mixture is diluted further with 0.9 % Sodium Chloride Injection or 5% Dextrose Injection before use. (Physicians' Desk Reference, 45th ed., 1991, pp. 1962-64, Medical Economics Company, Inc.) The macrolide molecule designated FK506, which has certain structural similarities to rapamycin, is also currently undergoing clinical investigation for inhibiting rejection in allograft organ transplantation in man. FK506 is isolated from *Streptomyces tsuskubaensis* and is described in U.S. Patent No. 4,894,366 to Okuhara et al., issued January 16, 1990 R. Venkataramanan et al., in Transplantation Proceedings, 22, No. 1, Suppl., 1 pp 52-56 (February 1990), report that the intravenous injectable formulation of FK506 is provided as a 10 mg/ml solution of FK506 in polyoxyethylated castor oil (HCO-60, a surfactant) and alcohol. The intravenous preparation must be diluted with saline or dextrose and administered as an infusion for 1 to 2 hours.

The Physicians' Desk Reference (45th ed., 1991, p. 2119, Medical Economics Company, Inc.) lists cyclosporine under the Sandimmune® tradename as available in 25 mg and 100 mg strength capsules and as an oral solution in 50 ml bottles. The 25 mg capsules contain 25 mg cyclosporine, USP, and alcohol, USP dehydrated, at a maximum of 12.7% by volume. The 100 mg capsules contain cyclosporine, USP, 100 mg and alcohol, USP dehydrated, at a maximum 12.7% by volume. Inactive ingredients in the oral capsules are corn oil, gelatin, glycerol, Labrafil M 2125 CS (polyoxyethylated glycolysed glycerides), red iron oxide, sorbitol, titanium dioxide, and other ingredients. The oral solution is available in 50 mg bottles containing cyclosporine, USP, 100 mg and Ph. Helv. alcohol at 12.5% by volume dissolved in olive oil, Ph. Helv./Labrafil M 1944 CS (polyoxyethylated oleic glycerides) vehicle which must be diluted further with milk, chocolate milk or orange juice before oral administration.

Azathioprine (available from Burroughs Wellcome Co., Research Triangle Park, N.C., under the tradename Imuran®) is another orally administered immunosuppressive agent prescribed alone or in conjunction with other immunosuppressive agents. The Physicians' Desk Reference (45th ed., 1991, pp. 785-787, Medical Economics Company, Inc.) lists azathioprine as 6-[1-methyl-4-nitroimidazol-5-yl)thio]purine, which is provided for oral administration in scored tablets containing 50 mg azathioprine and the inactive ingredients lactose, magnesium stearate, potato starch, povidone, and stearic acid.

Methods of drug delivery are designed to deliver an acceptable dosage of the medication to the patient. In the case of oral formulations, it is highly desirable to provide a dosage form which meets this criteria and which can be effectively administered, preferably self-administered, in either clinical or non-clinical situations. The present invention concerns formulations useful in the oral administration of rapamycin. Rapamycin has been shown to possess immunosuppressive, antifungal and antiinflammatory activity in vivo and to inhibit thymocyte proliferation in vitro. Therefore, these formulations are useful in the treatment of Candida albicans infections, diseases of inflammation and transplant rejection autoimmune diseases, including lupus, rheumatoid arthritis, diabetes melitus, multiple sclerosis, etc.

Because the formulations disclosed herein contain rapamycin, pharmaceutically acceptable salts of rapamycin, or combinations thereof, they are considered to have antitumor, antifungal and antiproliferative activities. As such, the formulations of this invention are useful in the treatment of transplantation rejection, such as heart, kidney, liver, bone marrow and skin transplants; autoimmune diseases such as lupus, rheumatoid arthritis, diabetes mellitus, myasthenia gravis and multiple sclerosis; diseases of inflammation such as psoriasis, dermatitis, eczema, seborrhea, inflammatory bowel disease and eye uveitis; solid tumors; fungal infections; and hyperproliferative vascular diseases, such as restenosis. The present invention, therefore, also provides formulations useful for inducing immunosuppression in a mammal in such need. Such inducements would comprise administering to said mammal an immunosuppressive amount of one or more of the formulations discussed herein.

The formulations of the present invention may be produced as a one component, ready to use solution of rapamycin in a non-aqueous system consisting of a solvent, surfactant and polyethylene glycol to produce an acceptable dosage form for chronic use in association with immunosuppressant therapy, as well as antitumor, antifungal and antiproliferative activities. The one component system may be adjusted to eliminate the solvent in cases where the drug concentration can be solubilized in the remaining ingredients. The invention may also be produced alternatively as a two component system either comprised of a dry component fill of 100% rapamycin and diluent or a drug concentrate and diluent.

Accordingly this invention provides a composition of matter comprising from about 0.05 to about 10.0 grams of rapamycin, per 100 ml composition, and a solvent system comprising from about 0.1 to about 25% by volume N,N-dimethylacetamide, from about 0.1 to about 10% by volume of surfactant, and from about 65 to about 99.8% by volume polyethylene glycol.

More particularly this invention provides a composition of matter comprising from about 0.1 to about 8.0 grams of rapamycin per 100 ml composition, and a solvent system comprising from about 0.5 to about 20% by volume N,N-dimethylacetamide, from about 0.5 to about 8% by volume of surfactant, and from about 72 to about 99% by volume polyethylene glycol. Also provided is a composition of matter comprising from about 0.5 to about 5.0 grams of rapamycin per 100 ml composition, and a solvent system comprising from about 1 to about 20% by volume N,N-dimethylacetamide, from about 1.0 to about 5% by volume of surfactant, and from about 75 to about 98% by volume polyethylene glycol.

In general, the formulations of the present invention include those comprised of a) rapamycin, b) N,N-dimethylacetamide (DMA), c) surfactant, and d) polyethylene glycol (PEG) 400 in the following concentrations (per 100 ml of formulation):

a) rapamycin at a concentration of from about 0.05 to about 10.0 grams per 100 ml:
b) DMA at a concentration of from about 0.1 to about 25 ml per 100 ml;
c) surfactant at a concentration of from about 0.1 to about 10 ml per 100 ml; and
d) PEG 400 at a concentration of from about 65 to about 99.8 ml per 100 ml.

More preferred formulations of the present invention include those in which the ingredients have the following ranges of concentration (per 100 ml formulation):

a) rapamycin at a concentration of from about 0.10 to about 8.0 grams per 100 ml:
b) DMA at a concentration of from about 0.5 to about 20 ml per 100 ml;

c) surfactant at a concentration of from about 0.5 to about 8 ml per 100 ml; and

d) PEG 400 at a concentration of from about 72 to about 99 ml per 100 ml.

The most preferred formulations of the present invention are those having the following ranges of concentrations (per 100 ml formulation):

a) rapamycin at a concentration of from about 0.5 to about 5.0 grams per 100 ml:

b) DMA at a concentration of from about 1 to about 20 ml per 100 ml;

c) surfactant at a concentration of from about 1 to about 5 ml per 100 ml; and

d) PEG 400 at a concentration of from about 75 to about 98 ml per 100 ml.

Examples of surfactants are polyoxyethylene sorbitol esters, polyoxyethylated fatty acids, polyoxyethylated fatty alcohols and polyoxyethylated glycerin hydroxy fatty esters.

More particulary surfactants that may be used with the present formulations include, but are not limited to, Polysorbate 20 (polyoxyethylene 20 sorbitan monolaurate), Polysorbate 60, Span 80® Sorbitan Oleate, a product of ICI Americas, Wilmington, DE, the Cremophor® surfactants produced by the BASF Corporation, Parsippany, NJ, and Polysorbate 80, which is defined by the Merck Index, 11th Edition, published by Merck & Co., Inc., Copyright 1989, on page 1254 as Sorbitan mono-9-octadecenoate poly(oxy-1,2-ethanediyl) derivatives, polyoxyethylene (20) sorbitan mono-oleate, Sorbitan mono-oleate polyoxyethylene, Sorlate, Tween 80, among others, and indicates an oleate ester of sorbitol and its anhydrides copolymerized with approximately 20 moles of ethylene oxide for each mole of sorbitol and sorbitol anhydrides. Polysorbate 80 is the surfactant preferred for use with the present invention.

The rapamycin dosage requirements for these formulations may vary depending upon the severity of the symptoms presented and the particular subject being treated. Projected daily oral dosages of the compounds of this invention, per kilogram of the patient's body weight, would be 0.005 - 75 mg/kg, preferably between 0.01 - 50 mg/kg, and more preferably between 0.05 - 10 mg/kg.

Treatment will generally be initiated with small dosages less than the optimum dose of the compound. Thereafter the dosage is increased until the optimum effect under the circumstances is reached. Precise dosages will be determined by the administering physician based on experience with the individual subject treated. In general, the formulations of this invention are most desirably administered at a concentration that will afford effective results without causing any harmful or deleterious side effects.

The present formulations may be administered to the patient by the means generally used for oral liquid medications. They may be taken, by themselves, or they may be dispersed in a liquid, such as water or juices. The formulations may also be capsulized, such as in starch capsules or soft elastic gelatin capsules. Rapamycin oral may be dispersed into water for dosing in the range of about 1 part of formula into about 9 parts water downward to about 1 part of formula into about 499 parts water by mixing for a minimum of about 60 seconds. This dispersion may be used over about a 1 hour period with mixing prior to dosing.

It is contemplated that when the formulations of this invention are used as an immunosuppressive or antiinflammatory agent, they can be administered in conjunction with one or more other immunoregulatory agents. Such other antirejection chemotherapeutic agents include, but are not limited to azathioprine, corticosteroids, such as prednisone and methylprednisolone, cyclophosphamide, cyclosporin A, FK-506, OKT-3, and ATG. By combining one or more of the formulations of the present invention with such other drugs or agents for inducing immunosuppression or treating inflammatory conditions, lesser amounts of each of the agents may be required to achieve the desired effect. The basis for such combination therapy was established by Stepkowski whose results showed that the use of a combination of rapamycin and cyclosporin A at subtherapeutic doses significantly prolonged heart allograft survival time. [Transplantation Proc. 23:507 (1991)].

It is also understood that the present formulations may be used with other ingredients used with conventional oral formulations such as, but not limited to, flavor enhancers, coloring agents, adjuvants, antifungal agents, antibacterial agents, etc.

Accordingly this invention also provides a process for preparing a composition of matter which comprises dissolving rapamycin in one or more components of a solvent system comprising from about 0.1 to about 25% by volume N,N-dimethylacetamide, from about 0.1 to about 10% by volume of surfactant, and from about 65 to about 99.8% by volume polyethylene glycol such that the concentration of rapamycin in the total composition is from about 0.05 to about 10.0 grams per 100ml.

The formulations of the present invention are exemplified, but not limited by, the preferred formulations and processes described below:

## EXAMPLE 1

The manufacture of rapamycin oral (Formula I) comprises adding rapamycin to the DMA and mixing until a solution results, adding the Polysorbate 80 to this mixture with mixing until clear and then bringing the sample to volume with polyethylene glycol (PEG) 400 and mixing until uniform. The sample is then filtered to remove particulates, packaged, and stored refrigerated and protected from light.

| Formula I: | |
|---|---|
| Ingredients | Amount |
| Rapamycin @ 100% | 5.0 gm |
| N,N-dimethylacetamide | 20.0 ml or 18.7 gm |
| Polysorbate 80, NF | 10.0 ml or 10.8 gm |
| Polyethylene Glycol 400, NF qs | 100 ml |

Manufacturing Directions: - One Component

1. Weigh rapamycin into a suitable container.
2. Add the N,N-dimethylacetamide to the container in Step #1. Mix until a solution results.
3. Add the Polysorbate 80 to the solution in Step #2. Mix until uniform.
4. QS to 100 ml with Polyethylene Glycol (PEG) 400. Mix sample until uniform.
5. Sterile filter sample through a 0.2 micron polytetrafluoroethylene (PTFE) filter.
6. Store sample refrigerated and protected from light.

Cynomolgus monkeys were administered orally Formulation I at a dose of 50 mg/kg of rapamycin and the following serum concentrations were determined at the indicated time after dosing.

## Rapamycin Concentration in Monkey Serum Dosed Orally with 50 mg/kg

Rapamycin Concentration ($\mu$g/ml)

Monkey No.

| Time | A | B | C | D | E |
|---|---|---|---|---|---|
| 0 | BDL | BDL | BDL | BDL | BDL |
| 1 hr | 0.063 | 0.025 | 0.014 | 0.020 | 0.150 |
| 2 hr | 0.078 | 0.030 | 0.006 | 0.040 | 0.061 |
| 3 hr | 0.078 | 0.047 | 0.005 | 0.016 | 0.077 |
| 4 hr | 0.022 | 0.048 | 0.006 | BDL | 0.105 |
| 6 hr | 0.025 | 0.109 | 0.006 | 0.028 | 0.015 |
| 9 hr | 0.019 | 0.020 | BDL | 0.006 | 0.017 |
| 12 hr | 0.014 | 0.032 | BDL | BDL | 0.020 |

BDL = Below detection limit (detection limit ~ or equal to 0.006 $\mu$g/ml)

EXAMPLE 2

The manufacture of rapamycin oral as a two component system may be achieved as in Formula II which represents a dry powder fill of rapamycin in one container and a diluent made by placing Polysorbate 80 into a suitable container, adding DMA with mixing until uniform and q.s. with PEG 400 or, alternatively, as in Formula III, by producing a drug concentrate in DMA, where rapamycin is solubilized by the DMA and a diluent system of Polysorbate 80 q.s. with PEG 400.

Formula II:

Part A

| Ingredients | Amount |
|---|---|
| Rapamycin @ 100 % | 5.00 gm |

Part B

| N,N-Dimethylacetatmide | 20.0 ml or 18.7 g |
|---|---|
| Polysorbate 80 | 10.0 ml or 10.8 g |
| Polyethylene Glycol 400 | q.s. 100 ml |

Manufacturing Directions:

Part A

1. Place the rapamycin into a suitable container.

Part B

1. Place the Polysorbate 80 into a suitable containiner.
2. Add the DMA to the Polysorbate 80 and mix until uniform.
3. QS with PEG 400 and mix until uniform.

Constitution Procedure:

1. Add the diluent from Part B to the rapamycin powder from Part A.
2. Mix until a solution results.
3. Filter sample.
4. Store refrigerated and protected from light.

Formula III:

Part A

| Ingredients | Amount |
|---|---|
| Rapamycin @ 100% | 2.5 gm |
| N,N-Dimethylacetamide | q.s. 10 ml |

6

Part B

| Polysorbate 80 | 5 ml |
|---|---|
| Polyethylene Glycol 400 | q.s. 40 ml |

Manufacturing Directions:

Part A

1. Weigh the rapamycin into a suitable container.
2. QS with DMA and mix until clear.
3. Place the concentrate into a suitably cleaned container and seal.

Part B

1. Place the Polysorbate 80 into a suitable containiner.
2. QS with PEG 400 and mix until clear.

Constitution Procedure:

1. Combine Parts A and B with mixing until homogeneous.
2. Sample may be filtered.
3. Store sample referigerated and protected from light.

## COMPARATIVE EXAMPLES

Rapamycin generally has poor water and oil solubility. It is only sparingly soluble in PEG-200 and 300 and is insoluble or very slightly soluble in commonly used aqueous co-solvent systems, such as, 20% ethanol/water, 10% DMA/water, 20% Cremophor EL®/water and 20% Polysorbate 80/water. It is insoluble in castor oil commonly used in oral systems. For these reasons, rapamycin cannot be readily placed into traditional systems such as the Sandimmune Oral and FK-506 Oral formulations presented below:

Sandimmune Oral

| Cyclosporine | 100 mg/ml |
|---|---|
| Ethanol | 23.5% V/V |
| Olive Oil/Labrafil M 1944CS | |

FK-506 Oral

| FK-506 | 20% |
|---|---|
| Methocellulose | |
| Water | |

Samples may be dispersed in water or encapsulated in a soft elastic gelatin capsule.

The oral formulations of the present invention have been found to provide higher blood levels of rapamycin after oral administration than traditional solutions, suspensions or emulsions previously used with non-water soluble drugs. Other examples of these traditional dosage forms, as applied to rapamycin, and their respective resulting blood levels are provided below:

**COMPARATIVE EXAMPLE 1**

Rapamycin Oral Suspension at 12.5 mg/ml

Formula:

| Ingredients | Amount |
|---|---|
| Rapamycin @ 100% | 1.25 gm |
| Ethanol | 10.0 gm |
| PEG 400 | 15.0 gm |
| Purified water | q.s. 100 ml |

Procedure:

1. Weigh the rapamycin into a suitably calibrated container.
2. Add the ethanol and mix until a solution results.
3. Add the PEG 400 and mix until clear.
4. While stirring sample in Step #3, q.s. to 100 ml with water.

Three Cynomolgus monkeys, listed below as A, B and C, were administered rapamycin oral suspension at a dose of 50 mg/kg of rapamycin and the following serum concentrations were determined at the indicated time after dosing.

## Rapamycin Concentration in Monkey Serum Dosed
## Orally with 50 mg/kg Rapamycin Oral Suspension

### Rapamycin Concentration (μg/ml)
### Monkey No.

| Time | A | B | C |
|---|---|---|---|
| 0 | BDL | BDL | BDL |
| 20 min | BDL | BDL | BDL |
| 40 min | BDL | BDL | BDL |
| 80 min | BDL | BDL | BDL |
| 3 hr | BDL | BDL | BDL |
| 6 hr | BDL | BDL | 0.006 |
| 12 hr | BDL | BDL | 0.013 |
| 24 hr | BDL | BDL | 0.039 |

BDL = Below detection limit (detection limit ~ or equal to 0.006 μjg/ml)

## COMPARATIVE EXAMPLE 2

Rapamycin Oral Solution at 25 mg/ml

Formula:

| Ingredients | Amount |
|---|---|
| Rapamycin @ 100% | 2.5 gm |
| Polysorbate 80 | 10.0 gm |
| Absolute Ethanol | 10.0 gm |
| Triacetin | q.s. 100 ml |

Procedure:

1. Weigh the Polysorbate 80 and ethanol into a suitable containiner.
2. QS with triacetin to 100 ml.
3. Add the rapamycin to the container in Step #2 and mix until a solution results.

Three Cynomolgus monkeys, listed below as A, B and C, were administered rapamycin oral solution at a dose of 50 mg/kg of rapamycin and the following serum concentrations were determined at the indicated time after dosing.

## Rapamycin Concentration in Monkey Serum Dosed Orally with 50 mg/kg Rapamycin Oral Solution

### Rapamycin Concentration (μg/ml)
### Monkey No.

| Time | A | B | C |
|---|---|---|---|
| 0 | BDL | BDL | BDL |
| 20 min | BDL | BDL | BDL |
| 40 min | BDL | BDL | BDL |
| 80 min | BDL | BDL | BDL |
| 3 hr | BDL | BDL | BDL |
| 6 hr | BDL | BDL | BDL |
| 12 hr | BDL | BDL | BDL |
| 24 hr | BDL | BDL | BDL |

BDL = Below detection limit (detection limit ~ or equal to 0.006 μg/ml)

## COMPARATIVE EXAMPLE 3

Rapamycin Oral Emulsion at 50 mg/kg

Formula:

| Ingredients | Amount |
|---|---|
| Rapamycin @ 100% | 5.0 gm |
| Dimethylacetamide | 10 ml |
| Olive Oil | q.s. 100 ml |

Procedure:

1. Place the rapamycin into a suitable container.
2. Add the dimethylacetatmide to the container in Step #1 and mix until clear.
3. QS with Olive Oil and mix until homogenous.

Three Cynomolgus monkeys, listed below as A, B and C, were administered rapamycin oral emulsion at a dose of 50 mg/kg of rapamycin and the following serum concentrations were determined at the indicated time after dosing.

### Rapamycin Concentration in Monkey Serum Dosed Orally with 50 mg/kg Rapamycin Oral Emulsion

#### Rapamycin Concentration (µg/ml)

#### Monkey No.

| Time | A | B | C |
|---|---|---|---|
| 0 | BDL | BDL | BDL |
| 20 min | BDL | BDL | BDL |
| 40 min | BDL | BDL | BDL |
| 80 min | BDL | BDL | BDL |
| 3 hr | BDL | BDL | BDL |
| 6 hr | BDL | 0.110* | BDL |
| 12 hr | BDL | BDL | BDL |
| 24 hr | BDL | BDL | BDL |

BDL = Below detection limit (detection limit ~ or equal to 0.006 µg/ml)

* assay result obtained from test lab appears aberent

## Claims

1. A composition of matter comprising from about 0.05 to about 10.0 grams of rapamycin, per 100 ml composition, and a solvent system comprising from about 0.1 to about 25% by volume N,N-dimethylacetamide, from about 0.1 to about 10% by volume of surfactant, and from about 65 to about 99.8% by volume polyethylene glycol.

2. A composition of matter comprising from about 0.1 to about 8.0 grams of rapamycin per 100 ml composition, and a solvent system comprising from about 0.5 to about 20% by volume N,N-dimethylacetamide, from about 0.5 to about 8% by volume of surfactant, and from about 72 to about 99% by volume polyethylene glycol.

3. A composition of matter comprising from about 0.5 to about 5.0 grams of rapamycin per 100 ml composition, and a solvent system comprising from about 1 to about 20% by volume N,N-dimethylacetamide, from about 1.0 to about 5% by volume of surfactant, and from about 75 to about 98% by volume polyethylene glycol.

4. A composition of matter according to Claim 1 which contains, per 100 ml of composition, 5.0 grams of rapamycin, 20 ml of N,N-dimethylacetamide, 10 ml of surfactant and polyethylene glycol 400 q.s. to 100 ml.

5 A multi-component product containing rapamycin and a solvent system as a combined preparation for mixing prior to oral administration to give a solution having a concentration of rapamycin from about 0.05 to about 10.0 grams per 100ml, said solvent system comprising, per 100 ml, from 0.1 to about 25 ml of N,N-dimethylacetamide, from about 0.1 to about 10 ml of surfactant and from about 65 to about 99.8 ml polyethylene glycol.

6 A multi-component oral rapamycin formulation comprising at least, in a first component, 5.0 grams of rapamycin and, in a second component, 20.0 ml of N,N-dimethylacetamide, 10.0 ml of surfactant and polyethylene glycol 400 q.s. to 100 ml.

7. A multi-component oral rapamycin formulation comprising at least, in a first component, 2.5 grams of rapamycin with N,N-dimethylacetamide q.s. to 10 ml and, in a second component, 5 ml of surfactant, and polyethylene glycol 400 q.s. to 40 ml.

8 A composition or formulation according to any one of Claims 1 to 6 in which the surfactant is one or more polyoxyethylene sorbitol esters, polyoxyethylated fatty acids, polyoxyethylated fatty alcohols and polyoxyethylated glycerin hydroxy fatty esters.

9. A process for preparing a composition of matter which comprises dissolving rapamycin in one or more components of a solvent system comprising from about 0.1 to about 25% by volume N,N-dimethylacetamide, from about 0.1 to about 10% by volume of surfactant, and from about 65 to about 99.8% by volume polyethylene glycol such that the concentration of rapamycin in the total composition is from about 0.05 to about 10.0 grams per 100ml.

EP 0 650 730 A1

| | European Patent Office | **EUROPEAN SEARCH REPORT** | Application Number EP 94 30 7118 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | WO-A-93 19763 (AMERICAN HOME PRODUCTS CORPORATION) * page 2, line 23 - page 5, line 19 * --- | 1-9 | A61K31/71 A61K9/08 A61K47/00 A61K47/10 A61K47/16 |
| X | TRANSPLANTATION, vol.51, no.1, 1991 pages 22 - 26 STANISLAW M. STEPKOWSKI ET AL. 'Rapamycin, a potent immunosuppressive drug for vascularized heart, kidney, and small bowel transplantation in the rat' * page 22, right column, paragraph 4 * ----- | 1-9 | |

TECHNICAL FIELDS SEARCHED    (Int.Cl.6)

A61K

The present search report has been drawn up for all claims

| Place of search | Date of completion of the search | Examiner |
|---|---|---|
| MUNICH | 24 January 1995 | Tzschoppe, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)